# EUROPEAN PATENT APPLICATION

(11) **EP 4 730 351 A1**
(43) Date of publication of application: **22.04.2026**
(21) Application number: 24306737.8
(22) Date of filing: 17.10.2024
(51) Int. Cl.: G16H 30/20, G16H 30/40, G16H 50/50

(54) **PREDICTING A CARDIOVASCULAR BEHAVIOR OF A NEWBORN TO BIRTH FROM THE FETUS PHYSIOLOGICAL PARAMETERS OF THE NEWBORN TO BIRTH**

(71) Applicant: Dassault Systèmes, 78140 Vélizy-Villacoublay (FR)
(72) Inventor: MORALES VARELA, Hernán, 78140 Vélizy-Villacoublay (FR); WEDER, Philipp Christoph, 78140 Vélizy-Villacoublay (FR)
(74) Representative: Bandpay & Greuter

(57) **Abstract**

The disclosure notably relates to a computer-implemented method for predicting a cardiovascular behavior of a newborn to birth from his/her fetus physiological parameters. The method comprises obtaining physiological parameters of the fetus, obtaining a non-calibrated surrogate cardiovascular model modeling a cardiovascular system of the fetus and modelling at least one physiological change triggered by the birth, calibrating the non-calibrated surrogate cardiovascular model with a data assimilation algorithm using the obtained physiological parameters of the fetus on the non-calibrated surrogate cardiovascular model, thereby obtaining a calibrated surrogate cardiovascular model of the fetus, and predicting the cardiovascular behavior of the newborn by triggering the at least one physiological change of the calibrated surrogate cardiovascular model of the fetus, thereby obtaining a calibrated surrogate cardiovascular model of the newborn.

## Description

### TECHNICAL FIELD

The disclosure relates to the field of computer programs and systems, and more specifically to a method, system and program for predicting a cardiovascular behavior of a newborn to birth from his/her fetus physiological parameters. The disclosure thus relates to the space of pediatric cardiology, particularly focused on congenital heart diseases.

### BACKGROUND

The transition from a fetal circulation or prenatal to a neonatal circulation is a strong event in the human life that significantly challenges the body. The main physiological changes - that are morphological and functional - of this transition include umbilical cord clamping, breathing, as well as strong pressure changes between systemic and pulmonary circulations that trigger valve and arterial closures to separate both systemic and pulmonary circulations. In most of the cases, the transition from prenatal to neonatal has no problems for the newborn.

However, in the presence of a congenital heart disease (CHD), this transition can lead to dead if unattended. Indeed, a CHD is a wide term for fetal heart malformations. Known malformations are, but not are limited to, the hypoplastic left ventricular syndrome, the complete transposition of the great arteries (also called dextro-transposition of the great arteries), the tetralogy of Fallot, the truncus arteriosus.

These malformations modify the normal heart physiology, as well as the pulmonary and systemic circulations, and therefore, once the fetal to neonatal transition arrives, the life of the child might be threated if the amount of flow, oxygen and nutrients are now well distributed.

An example of a critical scenario is the hypoplastic left heart syndrome (HLHS), where the left ventricle and surrounding cardiac structures are not well developed to sustain life. Under HLHS condition, the subject requires an urgent surgery to be performed within days.

Early detection of CHD is available through prenatal imaging, mainly with ultrasound (US). This detection is confirmed with postnatal US imaging, which can be complemented with either magnetic resonance imaging (MRI). These images can provide morphological and functional information of the subject. In the case of HLHS subject, morphological biomarkers such as the size of the aorta, left ventricle (LV), right ventricle (RV) and valve size are taken into account. Functional biomarkers include blood flow velocities with pulse or continuous wave Doppler US, as well as a qualitatively assessment with color Doppler US.

These examinations allow to confirm the detection of the malformation and to determine the severity of the subject. For example in subjects with HLHS, surgeons must perform the Norwood intervention (or equivalent) within days after birth (~4 days) since the heart of the newborn will not be capable to pump the required blood to the systemic circulation.

The decision to treat these subjects is difficult and not evident, since there are several elements to take into account, including the complexity of the procedure; its high risk of mortality; the irreversibility of the procedure (many structured are broken and rearranged on purpose); the patient's entire life is condemned to subsequent surgeries, regular hospital visit, medical examinations, etc. On top of that, the uncertainly to treat is very high, since in some cases, corrective or less destructive surgical procedures can be performed on the patient.

One problem is that both prenatal US imaging and postnatal US imaging require the subject to be substantially immobile, which is difficult (almost impossible) to obtain with a fetus or a newborn. Hence, the quality of the images may be not good (precise) enough for obtaining a clear view and identification of the malformation.

To support the medical team in the decision-making process, digital twins have risen as a supportive tool for physicians, as discussed for example in "A. Quarteroni, L. Dede', F. Regazzoni, and C. Vergara, "A mathematical model of the human heart suitable to address clinical problems," Jpn. J. Ind. Appl. Math., Apr. 2023, doi: 10.1007/s13160-023-00579-6."

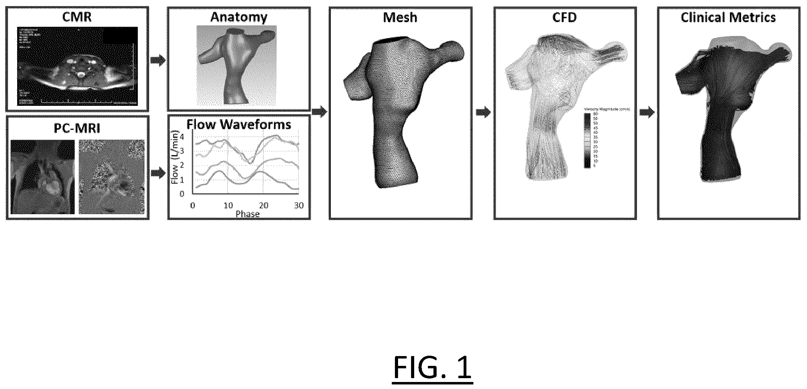

A digital twin can be constructed from the fetal, neonatal or adult stage, as discussed in "P. M. Trusty et al., "Fontan Surgical Planning: Previous Accomplishments, Current Challenges, and Future Directions," J Cardiovasc. Transl. Res., vol. 11, no. 2, pp. 133-144, Apr. 2018, doi: 10.1007/s12265-018-9786-0". In general, the digital twin is generated from anatomical and functional information coming from the patient, which is mainly collected from 3D images. A common pipeline to generate this 3D model is represented on **FIG. 1**. Inputs are Cardiac magnetic resonance (CMR) imaging used to create detailed pictures of the heart and arteries of the subject, and Phase contrast magnetic resonance (PC-MRI) imaging used to determine flow velocities. From these two inputs, a 3D mesh representing the heart and arteries of the subject is computed. Then, computational fluid dynamics (CFD) computations are used for obtaining a 3D model with velocity representation of blood flows. However, this method of construction of the virtual has several drawbacks. Firstly, a 3D, full-order digital twin for congenital heart disease planning in clinical routines requires time-consuming tasks, including several hours to days of manual preprocesses, as discussed in P. M. Trusty *et al.* These processes include image segmentation, registration and tracking with dedicated tools. Secondly, elevated computational resources have to be reserved for long periods, days-to-week of computation. In addition the calibration of the model also takes time to be adapted to the subject. In other words, it takes too long to obtain the model using this methodology so that the intervention cannot be carried out in the appropriate time window.

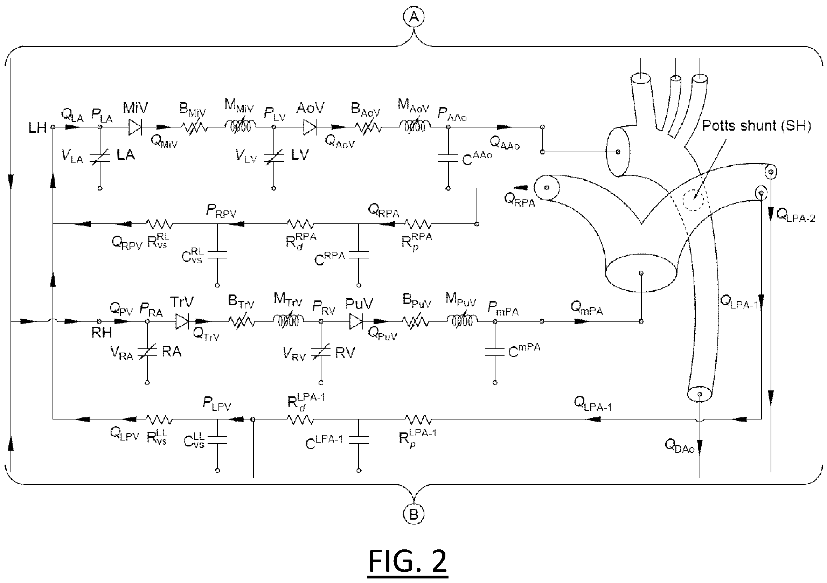

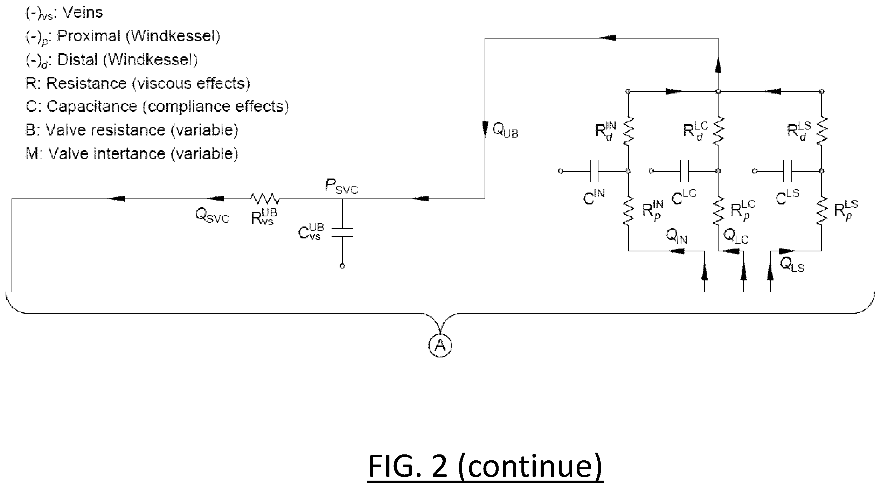

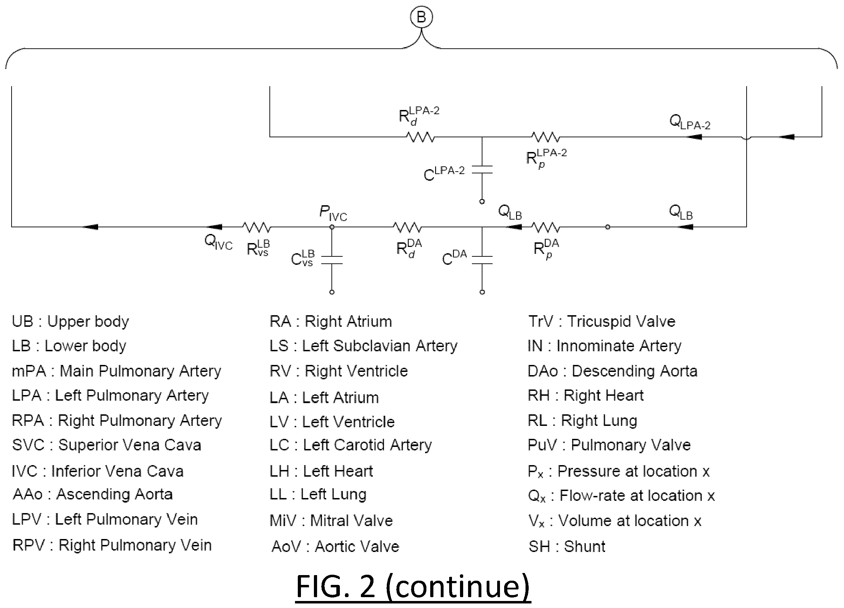

A way to alleviate these technical issues relies on surrogate models such as a 0D or lumped-parameter modeling (LPM), which is an efficient alternative to 3D. A 0D model of the cardiovascular system is a simplified representation of the components of the cardiovascular system, constructed by an equivalent set of electrical elements, such as resistance, capacitors, inductors, etc. **FIG. 2** depicts a 0D model in combination of a 3D model of a human circulation discussed in S. Pant et al., "Multiscale modelling of Potts shunt as a potential palliative treatment for suprasystemic idiopathic pulmonary artery hypertension: a paediatric case study," Biomech. Model. Mechanobiol., vol. 21, no. 2, pp. 471-511, Apr. 2022, doi: 10.1007/s10237-021-01545-2. The main advantage is the reduced computational expense compared to 3D models. But, as represented on **FIG. 2**, there are still 3D representations of parts of the cardiovascular system so that elevated computational resources are still requested. In addition, despite 0D model allows a flexible modeling of a patient's physiology and the fast simulation of the patient's circulatory system under different clinical conditions (e.g. prenatal vs. postnatal, preoperative vs. postoperative), as discussed in S. Pant et al., "Multiscale modelling of Potts shunt as a potential palliative treatment for suprasystemic idiopathic pulmonary artery hypertension: a paediatric case study," Biomech. Model. Mechanobiol., vol. 21, no. 2, pp. 471-511, Apr. 2022, doi: 10.1007/s10237-021-01545-2, S. Pant, C. Corsini, C. Baker, T.-Y. Hsia, G. Pennati, and I. E. Vignon-Clementel, "Inverse problems in reduced order models of cardiovascular haemodynamics: aspects of data assimilation and heart rate variability," J. R. Soc. Interface, vol. 14, no. 126, p. 20160513, Jan. 2017, doi: 10.1098/rsif.2016.0513 , S. Pant, C. Corsini, C. Baker, T.-Y. Hsia, G. Pennati, and I. E. Vignon-Clementel, "A Lumped Parameter Model to Study Atrioventricular Valve Regurgitation in Stage 1 and Changes Across Stage 2 Surgery in Single Ventricle Patients," IEEE Trans. Biomed. Eng., vol. 65, no. 11, pp. 2450-2458, Nov. 2018, doi: 10.1109/TBME.2018.2797999, C. D. Sá-Couto, P. Andriessen, W. L. Van Meurs, D. Ayres-De-Campos, and P. M. Sá-Couto, "A Model for Educational Simulation of Hemodynamic Transitions at Birth," Pediatr. Res., vol. 67, no. 2, pp. 158-165, Feb. 2010, doi: 10.1203/PDR.0b013e3181c2def3, the known literature focusses on generic modeling of the average patient or on the modeling of a specific aspect of a pathology. However, the use of digital twins in clinical routines requires a comprehensive pipeline for their construction and calibration and generic biomarkers to be evaluated by the clinician.

Within this context, there is still a need for an improved for predicting a cardiovascular behavior of a newborn to birth from his/her fetus physiological parameters.

### SUMMARY

It is therefore provided a computer-implemented method for predicting a cardiovascular behavior of a newborn to birth from his/her fetus physiological parameters. The method comprises:
- obtaining (A) physiological parameters of the fetus, in particular physiological parameters inferred from medical images;
- obtaining (C1) a non-calibrated surrogate cardiovascular model modeling a cardiovascular system of the fetus and modelling at least one physiological change triggered by the birth;
- calibrating (C2) the non-calibrated surrogate cardiovascular model with a data assimilation algorithm using the obtained physiological parameters of the fetus on the non-calibrated surrogate cardiovascular model, thereby obtaining (D1) a calibrated surrogate cardiovascular model of the fetus;
- predicting (E1) the cardiovascular behavior of the newborn by triggering the at least one physiological change of the calibrated surrogate cardiovascular model of the fetus, thereby obtaining a calibrated surrogate cardiovascular model of the newborn.

In examples, the method may comprise one or more of the following;
- the at least one physiological change triggered by the birth is selected among: a clamping of a placenta of the newborn; a breathing of the newborn; a closure of a foramen ovale of the newborn; and/or a closure of a ductus arteriosus of the newborn;
- before the obtaining (C1): obtaining (B) a generic non-calibrated surrogate cardiovascular model modeling a cardiovascular system of a general population of fetuses; and further comprising building the non-calibrated surrogate cardiovascular model modeling the cardiovascular system of the fetus by combining the obtained (B) generic non-calibrated surrogate cardiovascular model with the obtained (A) physiological parameters of the fetus, thereby the obtained non-calibrated surrogate cardiovascular model modeling the cardiovascular system of the fetus comprises at least all the obtained physiological parameters of the fetus.
- the general population of fetuses of the generic non-calibrated surrogate cardiovascular model comprises at least one cardiovascular behavior representative of a cardiovascular malformation, preferable one of the at least one cardiovascular behavior is representative of the cardiovascular malformation of the fetus;
- the wherein the obtained non-calibrated surrogate cardiovascular model modeling a cardiovascular system of the fetus is a lumped-parameter model;
- the data assimilation algorithm for calibrating (C1) is an Unscented Kalman Filter;
- extracting (D2), from the calibrated surrogate cardiovascular model of the fetus, predicted biomarkers of the fetus, each of the predicted biomarker providing a quantitative assessment of the biomarker;
- extracting (E2), from the calibrated surrogate cardiovascular model of the newborn, predicted biomarkers of the newborn, each of the predicted biomarker providing a quantitative assessment of the biomarker;
- computing, from the extracted (D2) biomarkers of the fetus and the extracted (E2) biomarkers of the newborn, a transition of each of the extracted biomarkers;
- the biomarkers are morphological biomarkers such as a size of the fetus and/or the newborn, a cardiac frequency, a diameter of an aorta, a size of a left ventricle, a size of a right ventricle, a valve size; and/or functional biomarkers such as a blood flow velocity;
- the non-calibrated surrogate cardiovascular model is a surrogate model, preferably the non-calibrated surrogate cardiovascular model is a 0D surrogate model;

It is further provided a computer-implemented method of use of the method according to the above method, for simulating a surgical intervention on a cardiovascular system of the fetus and/or the newborn. The method of use comprises inputting a physical change of the cardiovascular system of the fetus by modifying the calibrated surrogate cardiovascular model of the fetus and/or inputting a physical change of the cardiovascular system of the newborn by modifying the calibrated surrogate cardiovascular model of the newborn; performing again the predicting the cardiovascular behavior of the newborn.

It is also provided a computer program comprising instructions for performing the method and/or the method of use.

It is further provided a computer readable storage medium having recorded thereon the computer program
It is additionally provided a system comprising a processor coupled to a memory and a graphical user interface, the memory having recorded thereon the computer program.

### BRIEF DESCRIPTION OF THE DRAWINGS

Non-limiting examples will now be described in reference to the accompanying drawings, where:
- FIG. 1 shows a known example of a pipeline to generate a 3D model from image data;
- FIG. 2 shows a known example of a 0D model;
- FIG. 3 shows an example of a flowchart of the invention;
- FIG. 4 shows an example of 0D model of the cardiovascular system of the fetus;
- FIG. 5 shows an example of the 0D model of FIG. 4 with representation of physical changes triggered by the birth;
- FIG. 6 illustrates an example of use of unscented Kalman filter;
- FIG. 7 shows another example of a flowchart of the invention;
- FIG. 8 shows examples of different pathway of aberrant origin of the coronary artery arteries and their respective equivalent electrical circuit representations;
- FIGs. 9a and 9b show examples of predicted postnatal biomarkers;
- FIGs. 10a to 10c show examples of a transition from fetal to neonatal circulation in key vascular structures; and
- FIG. 11 shows an example of a computerized system.

### DETAILED DESCRIPTION

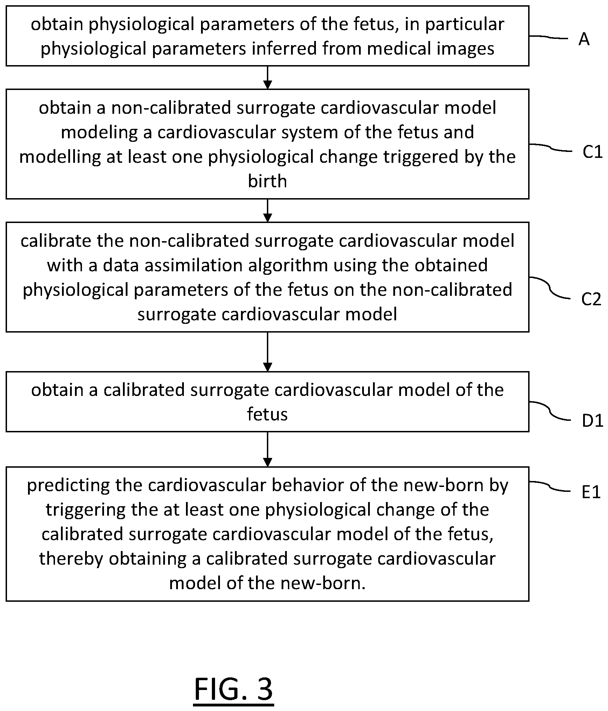

With reference to the flowchart of **FIG. 3**, it is proposed a computer-implemented method for predicting a cardiovascular behavior of a newborn to birth from his/her fetus physiological parameters.

"His/her" means that the fetus can be male or female, but an intersex fetus.

"Predicting a cardiovascular behavior" means that a quantitative statement that forecasts what will be observed under specific conditions, is provided. "Cardiovascular behavior" relates to actions or reactions of the heart and blood vessels, here of a newborn to birth.

"Newborn to birth" relates to the transition from the prenatal environment (of the fetus) to the external world at birth. This transition involves significant changes in several physiological systems, including the cardiovascular system. Regarding the fetal circulation, before birth, the fetus receives oxygen and nutrients from the mother through the placenta. Fetal circulation is characterized by certain unique physiological parameters, such as the presence of fetal shunts like the ductus arteriosus and foramen ovale, which allow blood to bypass the lungs since they are non-functional in the womb. At birth, several changes occur to facilitate the newborn's adaptation to breathing air and independent circulation: 1) the closure of fetal shunts: the ductus arteriosus and foramen ovale usually close within hours to days after birth as the newborn begins breathing and the lungs expand, allowing blood to flow into them for oxygenation. 2) Increase in pulmonary blood flow: With the onset of breathing, there is a rapid increase in blood flow to the lungs to facilitate oxygenation of blood. 3) Increase in systemic vascular resistance: as the newborn's lungs expand and oxygen levels rise, systemic vascular resistance increases, helping to establish separate pulmonary and systemic circulations. 4) Changes in blood pressure: there may be fluctuations in blood pressure as the cardiovascular system adjusts to the extrauterine environment. 5) Neonatal Circulation: after the transition period, the cardiovascular system of the newborn assumes a configuration similar to that of an adult, with blood flowing through the pulmonary and systemic circulations in parallel.

The cardiovascular system continues to mature over the next weeks and month of life.

"Physiological parameters" refers to a measurable characteristic or factor related to the function of an organism's body systems. Generally speaking, these parameters provide valuable information about the state of health, function, and balance within an organism. The physiological parameters of a fetus can vary throughout gestation and can be monitored to assess fetal well-being during pregnancy. Physiological parameters may include vital signs such as fetal Heart Rate (FHR), Fetal Oxygenation, Blood pressure.

Still in reference to **FIG. 3**, the method comprises obtaining **(A)** physiological parameters of the fetus, in particular physiological parameters inferred from medical images. Obtaining means that a system (e.g. a computerized system) executing the method can access (the data representative of) the physiological parameters that have otherwise been measured by known means, notably, but not only, by medical imaging techniques. The (data representative of) physiological parameters may be stored on a memory of the system for their use in the next steps of the method.

Examples of physiological parameters inferred from medical images are now discussed, being understood that they are not limited to these. Magnetic Resonance Imaging (MRI) can provide detailed images of different types of tissue based on their density and composition; it can differentiate between muscle, fat, and various organs based on their MRI signal characteristics. Dynamic contrast-enhanced MRI (DCE-MRI) can be used to assess blood flow and tissue perfusion in organs such as the brain, heart, and liver. Diffusion-weighted MRI (DW-MRI) measures the diffusion of water molecules within tissues. As another example of medical imaging technique, Ultrasound (US) Imaging can assess blood flow and velocity within blood vessel. For example, B-mode ultrasound, also known as brightness mode ultrasound, is a type of medical imaging technique that uses sound waves to produce real-time, two-dimensional images of the body's internal structures. For example, Color Doppler ultrasound is a type of medical imaging technique that combines traditional B-mode ultrasound imaging with Doppler ultrasound to visualize blood flow within the body's blood vessels. It provides real-time, color-coded images that depict the direction and velocity of blood flow, superimposed on the grayscale anatomical images obtained from the B-mode ultrasound. For example, PW-Doppler, or Pulsed Wave Doppler, is a specific mode of Doppler ultrasound used in medical imaging to measure blood flow velocities within blood vessels. Unlike Color Doppler, which provides information about blood flow direction and velocity over a wide area, PW-Doppler allows for precise measurements of blood flow at a specific location along the vessel.

The physiological parameters of the fetus may also include, but are not limited to, the gestational age of the fetus, a weight estimation, size, etc. Some of these parameters may be obtained from imaging techniques too.

Next, is obtained **(C1)** a non-calibrated surrogate cardiovascular model modeling a cardiovascular system of the fetus and modelling at least one physiological change triggered by the birth. Obtaining means that a system (e.g. a computerized system) executing the method can access (the data representative of) the non-calibrated surrogate cardiovascular model modeling a cardiovascular system of the fetus. The (data representative of) non-calibrated surrogate cardiovascular model modeling a cardiovascular system of the fetus may be stored on a memory of the system for their use in the next steps of the method. The non-calibrated surrogate cardiovascular model is a simplified representation of the cardiovascular system of the fetus that does not rely on specific calibration procedures or patient-specific data. Instead of precisely modeling individual physiological parameters such as blood pressure, heart rate, or vascular resistance for the fetus, the non-calibrated surrogate cardiovascular model of the fetus uses generic or population-based parameters of fetuses to simulate cardiovascular dynamics.

The non-calibrated surrogate cardiovascular model modeling a cardiovascular system of the fetus is a surrogate model, that is, a simplified and faster computational model that represents the cardiovascular system of the fetus. In an example, the non-calibrated surrogate cardiovascular model, which is a surrogate model, may be a 0D model. A 0D model is a surrogate model of a high-fidelity model without spatial dimension and with parameters describing the overall system dynamics, such as blood flow rate, pressure and volume. A high-fidelity model is a complex and complete 3D model. In another example, the non-calibrated surrogate cardiovascular model may be an Artificial Intelligence-based model that can replace a 0D model. An example of an Artificial Intelligence-based model is a trained algorithm that learn from past experience, which come from the solution of one or multiple high-fidelity models as dsocussed in P. Vurtur Badarinath, M. Chierichetti, and F. Davoudi Kakhki, "A Machine Learning Approach as a Surrogate for a Finite Element Analysis: Status of Research and Application to One Dimensional Systems," Sensors, vol. 21, no. 5, p. 1654, Feb. 2021.

In examples, the 0D model is a lumped-element model, which is a simplified representation of the cardiovascular system of the fetus that assumes all components are concentrated at a single point and their behaviour can be described by idealized mathematical models. Lumped-element models are known in the art.

In the context of the cardiovascular system of the fetus, the lumped-parameter model is used to represent the complex network of blood vessels, valves, and the heart as a simplified system with discrete compartments or "lumped" elements. Each element represents a group of similar components with similar behaviour. The entire systemic circulation can be represented by several compartments. For example, an arterial compartment can represent a set of large arteries such as the descending aorta, iliac and femoral arteries. As another example, a capillary compartment represents the network of tiny blood vessels where exchange of nutrients and gases occurs. Further example, a venous compartment represents the large veins where blood returns to the heart. In these examples, each compartment is characterized by parameters such as compliance (the ability to stretch), resistance (the hindrance to flow), and capacitance (the ability to store blood). It is to be understood that the lumped-parameter model of the cardiovascular system of the fetus can comprises many further types of compartments.

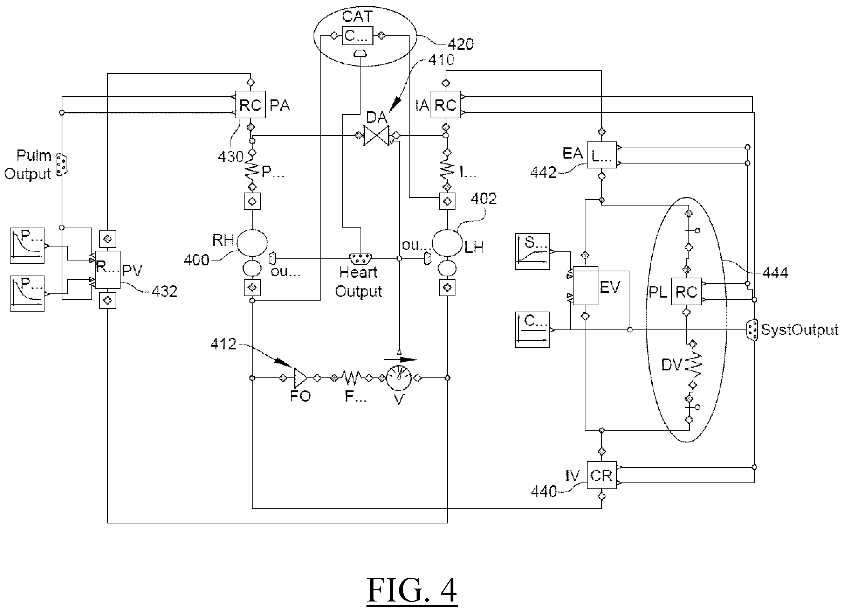

**FIG. 4** shows an example of a lumped-parameter model of the cardiovascular system of the fetus. This generic prenatal digital twin architecture has been modeled with Dymola^{™}, which is a commercial software tool published by Dassault Systèmes^{™} used for modeling and simulation of dynamic systems. This example of the lumped-parameter model has the capability to simulate the birth transition. In the middle of the figure, the sphere **400** represents the right heart ventricle, and the sphere **402** represents the left heart ventricle. The arrow **410** points to the ductus arteriosus (DA), and the arrow **412** points to the foramen ovale (FO). The model further comprises the coronary tree **420** that supplies the heart muscle. On the left of the figure, is modeled the respiratory circulation with both pulmonary arteries (PA) **430** and pulmonary veins **(PV) 432** blocks. On the right of **FIG. 4**, the systemic circulation is modeled (the coronary tree **420** is on top of the figure) with the intrathoracic veins **440,** the extrathoracic veins **442.** The systemic circulation also includes the placenta (PL) **444.**

Still in reference to **C1**, at least one physiological change triggered by the birth is modelled. At **C1**, a change that is modelled is not applied yet on the non-calibrated surrogate cardiovascular model modeling a cardiovascular system of the fetus, but it is stored, e.g. on a storage medium so that it can be applied for a next step of the method, as it will be discussed after.

The physiological changes triggered by the birth starts with the onset of labour and are essential for the transition from intrauterine to extrauterine life, in the case of a normal birth.

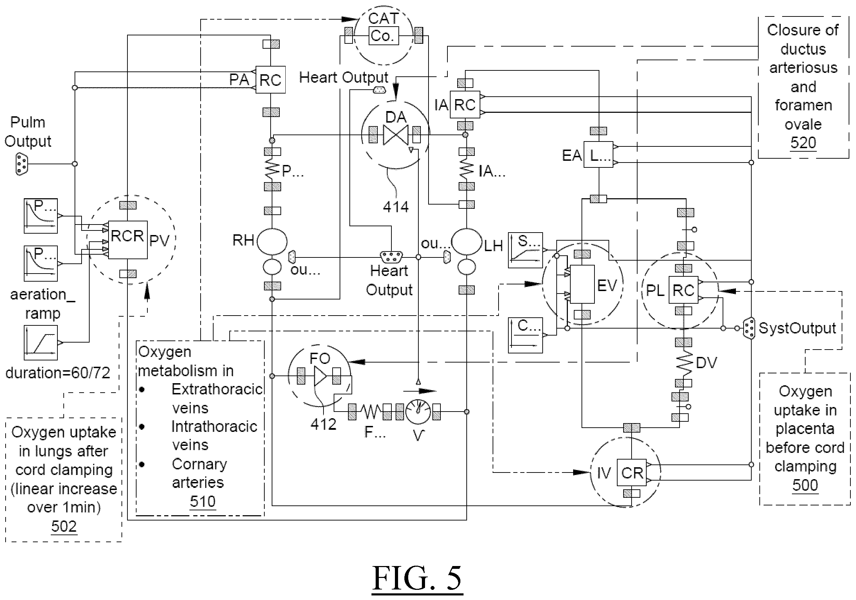

Examples of physiological changes triggered by the birth are now discussed. In an example, the clamping of a placenta of the newborn may be modeled. The clamping of a placenta of the newborn is a common procedure during childbirth and is generally performed by clamping the umbilical cord, which connects the newborn to the placenta. Referring now to the example of **FIG. 5**, is represented the model of **FIG. 4** on which the modeling of the examples of physiological changes triggered by the birth have been applied. The clamping of the placenta triggers the total suppression of the flow on the right branch **444** (surrounded by the ellipse) on FIG. 4. Therefore, the modeling of the clamping of the placenta of the newborn comprises, in this example, the closing of the right branch **444.**

In an example, a physiological change triggered by the birth may comprise the breathing of the newborn. The gas exchange in the lungs is activated. On the example of **FIG. 5**, oxygen uptake is changed from the placenta **444** - oxygen uptake is performed in placenta before the cord clamping- to the lungs **432** - oxygen uptake is performed in the lungs after the cord clamping- with, for example, a linear increase over a time period of one minute. Therefore, the modeling of the breathing of the newborn comprises activation of the element lung **432** with a linear increase of the oxygen, for instance, introduced in the cardiovascular system of the newborn (e.g. from 0% to 100%) over approximately one minute.

In an example, a physiological change triggered by the birth may comprise the closure of the foramen ovale of the newborn. On the example of **FIG. 5**, the closure of the segment **412** in the circuit expresses this physiological change, which is controlled by the pressure difference between the left and right atria. Therefore, the modeling of closure of the foramen ovale of the newborn comprises closing the segment **412** in the non-calibrated surrogate cardiovascular model modeling the cardiovascular system of the fetus.

In an example, a physiological change triggered by the birth may comprise the closure of the ductus arteriosus of the newborn. This change may be expressed by a signal that decreases the arterial diameter, thus increasing its resistance. On the example of **FIG. 5**, the closure of the ductus arteriosus of the newborn is activated with the element **414** of the circuit that represents the decrease of the arterial diameter.

The examples of physiological changes have been presented separately, being understood that any combination of two or more of these examples may be performed at the at least one physiological change triggered by the birth is modelled **(C1)**.

Back to **FIG. 3**, at **C2**, the non-calibrated surrogate cardiovascular model is calibrated with a data assimilation algorithm using the obtained physiological parameters of the fetus on the non-calibrated surrogate cardiovascular model. The non-calibrated surrogate cardiovascular model is adapted to capture the desirable components of the cardiovascular system of the fetus. The functional data, that is the obtained physiological parameters of the fetus, are used for that purpose.

The obtained physiological parameters of the fetus comprise all the numerical model parameters, e.g., resistances or elasticities that are defined in the non-calibrated surrogate cardiovascular model. The calibration thus makes the digital twin to reproduce the fetus (prenatal) physiological state. To achieve this objective, a data assimilation algorithm is used. Data assimilation algorithms, as known in the art, combine observations (the obtained physiological parameters of the fetus) with the non-calibrated surrogate cardiovascular mode to produce estimates of the state of non-calibrated surrogate cardiovascular model; a calibrated surrogate cardiovascular model of the fetus will be obtained as a result of the calibration.

Numerous data assimilation methods exist in the literature, including variational, sequential, and deep learning methods, as discussed in R. W. May et al., "From fetus to neonate: A review of cardiovascular modeling in early life," WIREs Mech. Dis., p. e1608, Mar. 2023, doi: 10.1002/wsbm.1608. Any of these methods can be used with the present invention.

In an example, the data assimilation algorithm for calibrating the non-calibrated surrogate cardiovascular model is an Unscented Kalman Filter (UKF), as discussed for example in E. A. Wan and R. Van Der Merwe, "The unscented Kalman filter for nonlinear estimation," in Proceedings of the IEEE 2000 Adaptive Systems for Signal Processing, Communications, and Control Symposium (Cat. No.00EX373), Lake Louise, Alta., Canada: IEEE, 2000, pp. 153-158. doi: 10.1109/ASSPCC.2000.882463. The Unscented Kalman Filter is a sequential and recursive algorithm that assimilates a dynamic system to noisy, observed time series data. It is an adaptation of the classical Kalman filter to non-linear models. This is particularly adapter to the cardiovascular system as cardiovascular lumped parameter models are non-linear.

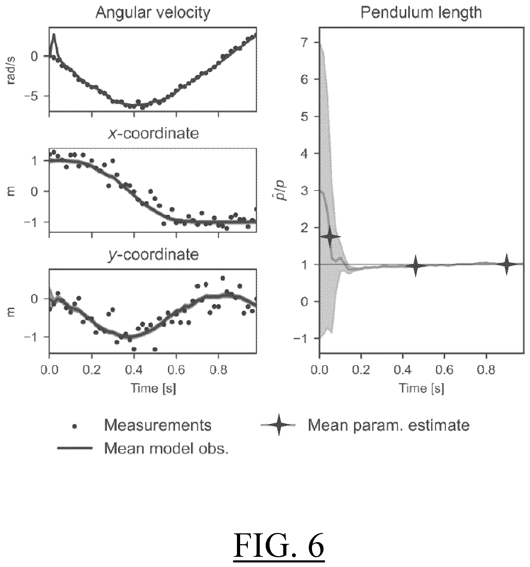

**FIG. 6** shows the results of applying the UKF to an equivalent but simplified inverse problem (only one variable) where the length of a pendulum is found (on the right plot). In **FIG. 6**, the angular velocity, the x and y coordinates are measurements represented by the dots on the left plots. These measurements were generated by a mathematical model represented by the continuous line (on the left plots) after adding some noise. This is a very simple example, and the procedure is the same when the UKF is applied to calibrate a cardiovascular system. The main difference is that the number of parameters to be calibrated is ~200.

In an example, a specialized versions of the UKF for cardiovascular applications able to take into account different heart rates from several medical examinations (S. Pant, C. Corsini, C. Baker, T.-Y. Hsia, G. Pennati, and I. E. Vignon-Clementel, "Inverse problems in reduced order models of cardiovascular haemodynamics: aspects of data assimilation and heart rate variability," J. R. Soc. Interface, vol. 14, no. 126, p. 20160513, Jan. 2017, doi: 10.1098/rsif.2016.0513) may be used, by filtering in the frequency domain (L. O. Muller, A. Caiazzo, and P. J. Blanco, "Reduced-Order Unscented Kalman Filter With Observations in the Frequency Domain: Application to Computational Hemodynamics," IEEE Trans. Biomed. Eng., vol. 66, no. 5, pp. 1269-1276, May 2019, doi: 10.1109/TBME.2018.2872323), reduced-order formulations, and sensitivity analysis procedures (A. Caiazzo, F. Caforio, G. Montecinos, L. O. Muller, P. J. Blanco, and E. F. Toro, "Assessment of reduced-order unscented Kalman filter for parameter identification in 1-dimensional blood flow models using experimental data: Kalman filter for 1D blood flow models and in vitro data," Int. J. Numer. Methods Biomed. Eng., vol. 33, no. 8, p. e2843, Aug. 2017, doi: 10.1002/cnm.2843), to optimize the filter's performance.

The calibration having been performed, at **D1**, a surrogate cardiovascular model of the fetus is obtained (thus available for further steps of the method). Thus **D1** corresponds to the calibrated prenatal digital twin, which architecture is show in **Fig. 4**. From this calibrated surrogate cardiovascular model of the fetus, prenatal biomarkers may be delivered to the medical team for evaluation, e.g. the state of the cardiovascular system of the fetus. These prenatal biomarkers are already valuable for the physicians since there represent a quantitative assessment (and not mainly qualitative as known in the art) of the patient health condition and can provide variables that are not directly measured from the fetus, such as the oxygen saturation and flow rates.

Still in reference to **FIG. 3**, at **E1**, the cardiovascular behavior of the newborn is predicted by triggering at least one physiological change of the calibrated surrogate cardiovascular model of the fetus. Triggering at least one physiological change means that the obtained calibrated surrogate cardiovascular model of the fetus is modified to reflect the model of at least one physiological change (A). Examples of physiological changes triggered by the birth have been discussed. Triggering the at least one physiological change means that the calibrated surrogate model is modified by applying the model(s) of the physiological change(s), e.g. the parameters of the calibrated surrogate cardiovascular model) are modified with new values provided by the model(s) of the at least one physiological change triggered by the birth.

As a result of the triggering the change(s), a calibrated surrogate cardiovascular model of the newborn is obtained. Thus, from the prenatal digital twin, the birth of the fetus can be simulated, and his/her postnatal digital twin has been produced.

The present method thus proposes the generation of a personalized digital twin of a subject at prenatal age (that is, for a fetus), capable of predicting the neonatal (post-birth) physiological condition, e.g. to warn of a non-viable scenario after birth. For that, subject data (medical imaging and non-image-derived data) will be collected (A) from the subject at the fetal stage. Then, a digital twin of the cardiovascular system of the fetus will be calibrated to the patient data using data assimilation algorithm. Once the digital twin is personalized, the neonatal physiological condition is predicted by simulating the birth of the patient through his/her digital twin. This post-birth simulation is able to provide the postnatal physiological conditions, such as oxygen saturation in main/critical vessels (aorta, coronaries, and veins for instance). In this way, if the subject has a post-birth condition that compromises his/her survival, then it is possible to plan all the needed measures to ensure the post-birth viability of the subject before birth. Moreover, this digital twin reduces the subjective interpretation of the patient data analysis and provides a platform where physicians can simulate (tests by simulations) different therapeutic strategies by combining pre and postnatal stages, as discussed hereinbelow.

Such a method improves the predictions of the cardiovascular behavior of the newborn to birth from his/her fetus physiological parameters. Notably, the method provides a quantitative assessment of the subject condition, instead of a qualitative interpretation of a medical specialist. This quantification reduces the required time to comprehend the state of the cardiovascular system of the subject, increases confidence of for any decision to be made by a medical team, and importantly, provides additional information aiming at the best treatment for a given subject. The method can be compared as a tool to generalize to identify and understanding CHD in general, making easier comparisons among subject's conditions. For example, the present method according to the invention can lead to a successful corrective intervention, rather than a palliative/destructive surgery (such as the Norwood intervention), reducing the subject suffering to additional interventions and medical visits, as well as reducing costs. Furthermore, the choice of a 0D model keeps simulation times short enough to evaluate multiple scenarios under time pressure. It is to be understood that the present method only proposes indications and options, the choice of using them remaining at the free discretion of the physician.

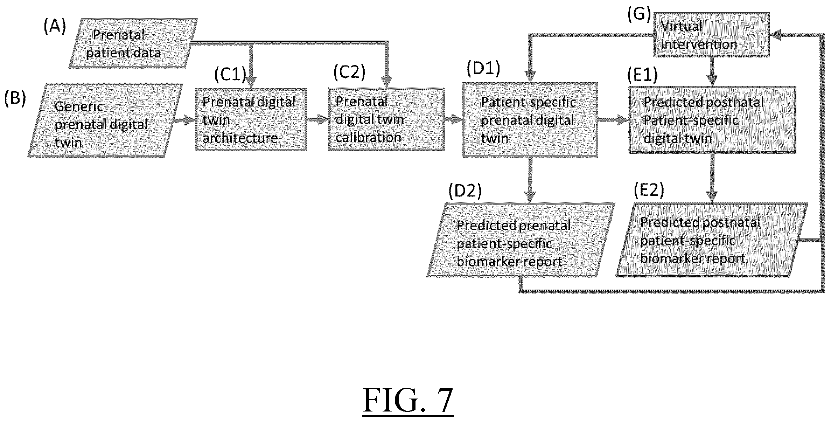

Further examples of the method are now discussed in reference to **FIG. 7. FIG. 7** is a combination of these further examples, being understood that these examples may be individually combined with the method of **FIG. 3**, or any combination of these examples may be combined with the method of **FIG. 3****.**

In examples, the non-calibrated surrogate cardiovascular model modeling a cardiovascular system of the fetus is obtained from a **(B)** generic non-calibrated surrogate cardiovascular model modeling a cardiovascular system of a general population of fetuses and the obtained **(A)** physiological parameters of the fetus.

The generic non-calibrated surrogate cardiovascular model models a cardiovascular system of a general population of fetuses. This means that the generic non-calibrated surrogate cardiovascular model comprises all the discrete compartments or "lumped" elements required for modeling a non-calibrated surrogate cardiovascular model for the given general population of fetuses.

The genericity of the generic non-calibrated surrogate cardiovascular model thus depends on the definition of the general population of fetuses. In an example, the general population of fetuses may be validated on population data to reflect an average subject, such as for example in C. D. Sá-Couto, P. Andriessen, W. L. Van Meurs, D. Ayres-De-Campos, and P. M. Sá-Couto, "A Model for Educational Simulation of Hemodynamic Transitions at Birth," Pediatr. Res., vol. 67, no. 2, pp. 158-165, Feb. 2010, doi: 10.1203/PDR.0b013e3181c2def3, or in A. G. Munneke, J. Lumens, and T. Delhaas, "Cardiovascular fetal-to-neonatal transition: an in-silico model," Pediatr. Res., vol. 91, no. 1, pp. 116-128, Jan. 2022, doi: 10.1038/s41390-021-01401-0. In another example, the generic model may be restricted to one or more CHD ; the general population of fetuses is restricted to a population of fetuses suffering of the said one or more CHD. Restricting the population of fetuses allows to obtain a generic non-calibrated model that is closer to the physiological reality of the subject. Especially, obtaining a generic non-calibrated model of the cardiovascular system of a restricted population of fetuses (e.g. fetuses suffering of at least one (one or one of more) CHD) improves the process of calibration.

The generic non-calibrated surrogate cardiovascular model being obtained, the non-calibrated surrogate cardiovascular model of the fetus is built by combining the obtained (B) generic non-calibrated surrogate cardiovascular model with the obtained (A) physiological parameters of the fetus. The building allows obtaining a simplified mathematical representation of the cardiovascular system of the fetus, e.g. obtaining a simplified mathematical representation of the cardiovascular system of the fetus that may comprise a specific morphological malformations of the fetus, depending on the selected general population of fetuses as discussed previously.

In example, the building may comprise performing manual, semi or automatically modifications of the obtained (B) generic non-calibrated surrogate cardiovascular model, depending on the acquired images and/or patient-specific data available. For example, it is possible to automatically label and segment valves and cardiac chambers from ultrasound images, as shown in documents T. Kim, M. Hedayat, V. V. Vaitkus, M. Belohlavek, V. Krishnamurthy, and I. Borazjani, "Automatic segmentation of the left ventricle in echocardiographic images using convolutional neural networks," Quant. Imaging Med. Surg., vol. 11, no. 5, pp. 1763-1781, May 2021, doi: 10.21037/qims-20-745; P. Carnahan, J. Moore, D. Bainbridge, M. Eskandari, E. C. S. Chen, and T. M. Peters, "DeepMitral: Fully Automatic 3D Echocardiography Segmentation for Patient Specific Mitral Valve Modelling," in Medical Image Computing and Computer Assisted Intervention - MICCAI 2021; B. Yuan et al., "Automatic valve segmentation in cardiac ultrasound time series data," in Medical Imaging 2018: Image Processing, E. D. Angelini and B. A. Landman, Eds., Houston, United States: SPIE, Mar. 2018, p. 69. doi: 10.1117/12.2293255 ;A. Arafati et al., "Generalizable fully automated multi-label segmentation of four-chamber view echocardiograms based on deep convolutional adversarial networks," J. R. Soc. Interface, vol. 17, no. 169, p. 20200267, Aug. 2020, doi: 10.1098/rsif.2020.0267.

As another example, if a particular physiological parameter of the subject is not present in the generic model, such as for example the origin of coronary arteries, which can widely variate, a predefined set of arterial configurations may be at disposal of the user for modifying the generic non-calibrated surrogate model. From this set of arterial configurations, the user can select the closest representation of the subject's arterial tree and plug it into the main architecture. For instance, in the case of the coronaries, this is done by replacing the corresponding generic coronary block by the chosen coronary arterial tree in the set of arterial configurations. Another way to build specific arterial segments with unique branches and/or duplicated arteries is by drawing the connections, as can be done with Dymola^{™}.

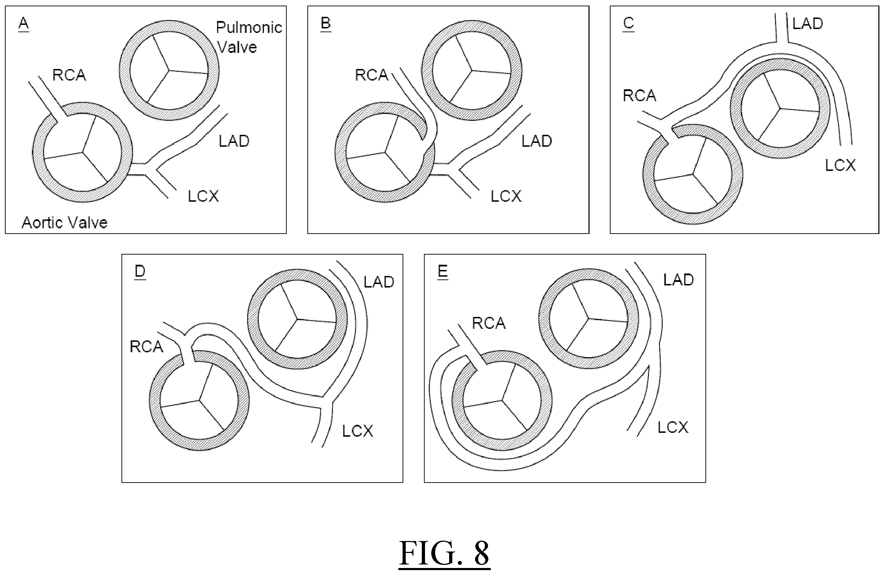

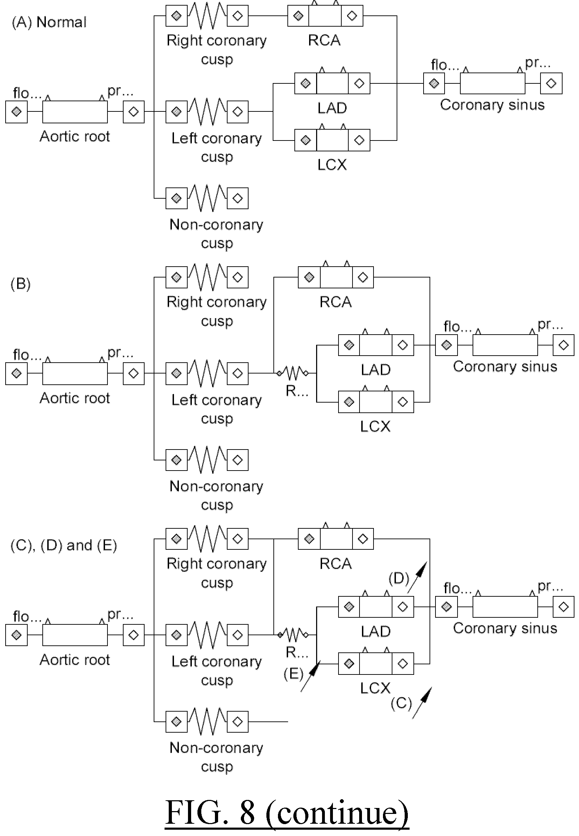

Referring to **FIG. 8**, examples of predefined set of arterial configurations that may be at disposal of the user are represented. Each configuration shows a different pathway of the coronary arteries, where RCA is the right coronary artery, LAD the left anterior descending artery and LCX the left circumflex artery. The physiological configuration noted A represents a normal anatomy; its equivalent electrical circuit representation is shown on the top right of the figure. The physiological configuration noted B represents an RCA from left coronary cusp, inter-arterial course; its equivalent electrical circuit representation is in the middle of the right of the figure. The physiological configuration noted C represents a left main (LM) from right coronary cusp, anterior course. The configuration noted D represents a LM from right coronary cusp, inter-arterial course, and the representation noted E represents a LM from right coronary cusp, posterior course. These three configurations C, D and E have the equivalent electrical circuit representation that is shown in the bottom right of the figure; the arrows represent the predominant resistance with respect to the other two arterial segments to catch configurations C, D and E.

Referring back to **FIG. 4**, the model comprises the coronary tree **420** that supplies the heart muscle. In the event the fetus subject has a CHD that is not represented in the generic non-calibrated surrogate cardiovascular model, the user can modify the model with one the arterial configuration of the predefined set of arterial configurations of **FIG. 8** for adapting the generic model. For example, if the fetus has the malformation represented by the physiological configuration noted C, the coronary tree **420** of the generic model may be replaced by the equivalent electrical circuit representation of the physiological configuration noted C, as shown in **FIG. 8****.**

Hence, the obtained non-calibrated surrogate cardiovascular model modeling the cardiovascular system of the fetus comprises at least all the physiological parameters of the fetus observed from the physiological parameters of the fetus. If the fetus comprises a malformation, e.g. a CHD malformation, then non-calibrated surrogate cardiovascular model models the physiological parameters of the malformation of the fetus.

As said hereinabove, the genericity of the generic non-calibrated surrogate cardiovascular model depends on the definition of the general population of fetuses. In examples, the general population of fetuses is selected based on a specific physiological parameters of the fetus. For example, the general population may be restricted to fetuses with at least one cardiovascular behavior that is representative of a cardiovascular malformation of the fetus' subject. In this situation, the generic non-calibrated surrogate model is adapted for one (or at least one) physiological malfunction, e.g., a CHD.

Referring back to the example of **FIG. 7**, are now discussed examples that concern the blocks noted **D2**, **E2** and **G**.

In examples, predicted biomarkers of the fetus are extracted **D2** from the calibrated surrogate cardiovascular model of the fetus. A biomarker is a measurable biological feature of a biological system. Examples of biomarkers in humans are weight, size, the amount of a specific molecule in blood, blood pressure, etc.

In examples, the extracted biomarkers may be morphological biomarkers. Morphological biomarkers refer to physical characteristics or features of biological entities that can be objectively measured, quantified, and analyzed. These biomarkers are often related to the structure, shape, size, or appearance of cells, tissues, organs, or organisms. Morphological biomarkers may be, but are not limited to, a size of the fetus and/or the newborn, a cardiac frequency, a diameter of an aorta, a size of a left ventricle, a size of a right ventricle, a valve size.

In examples, the extracted biomarkers may be functional biomarkers. Functional biomarkers provide insights into the physiological or functional status of biological systems. They reflect the dynamic processes or activities occurring within cells, tissues, organs, or organisms. Functional biomarkers may be, but are not limited to, a blood flow velocity.

A predicted biomarker thus provides a quantitative assessment of the biomarker, and the quantitative assessment may be done for a given point in time. One understands that the extraction of the predicted biomarker is performed on the obtained calibrated surrogate cardiovascular model of the fetus **(D1)**, as shown on **FIG. 7**. The extraction of the predicted biomarker is performed as known in the art. For example, if the obtained calibrated surrogate cardiovascular model of the fetus is 0D model, e.g. a lumped-parameter model, the input may be scalar value(s) (single-dimensional inputs, typically represented by a single value) that are passed through the model in order to obtain an output, the extracted biomarkers.

In examples, predicted biomarkers of the newborn are extracted **E2** from the calibrated surrogate cardiovascular model of the newborn. This is performed in a similar manner as for the extraction of the biomarkers of fetus and the comments apply.

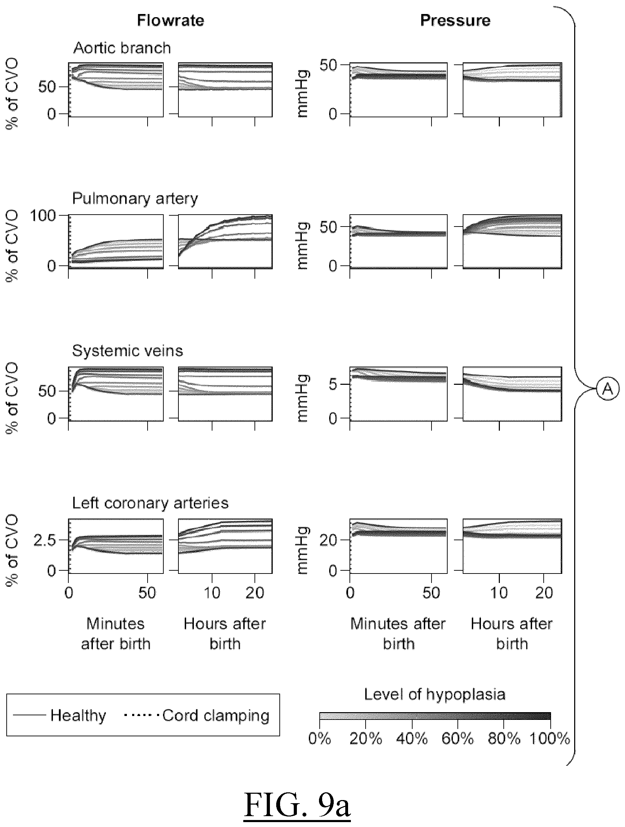

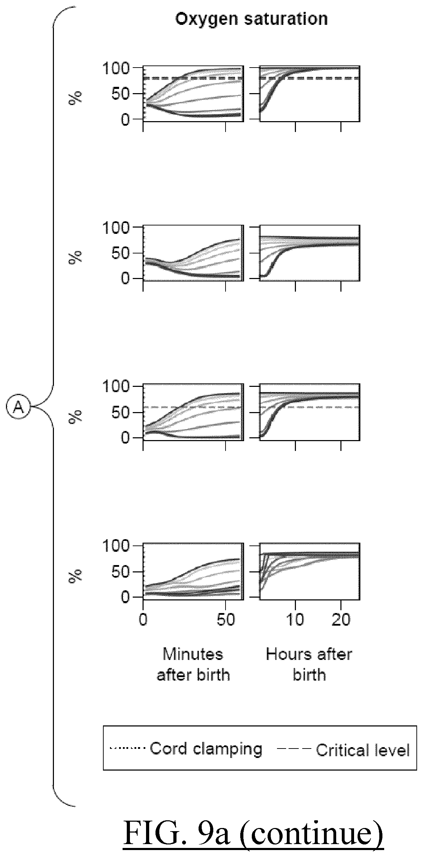

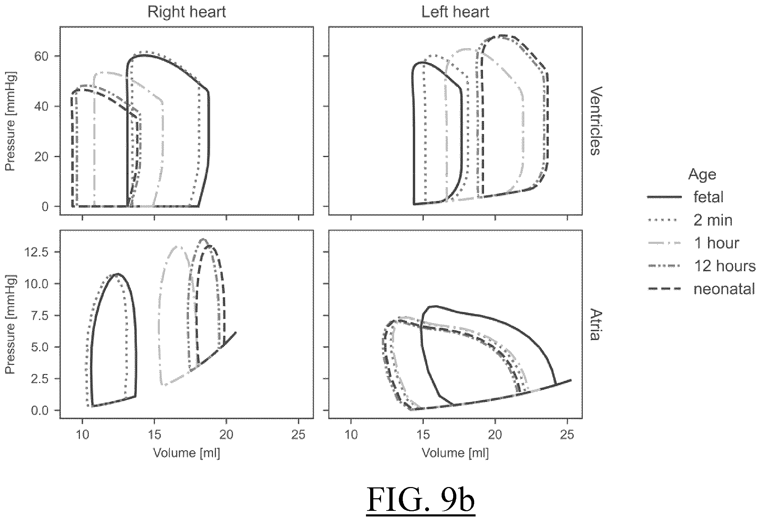

In an example, predicted biomarkers of the fetus **(E1)** and the predicted biomarkers of the newborn **(E2)** are combined for computing a transition of each of the extracted biomarkers. Computing the transition of each of the extracted biomarkers involves that an identical biomarker applies for both the fetus and the newborn. **FIGs. 9a** and **9b** shows examples of combined reports, where the flow rate, oxygen saturation are depicted. On **FIG. 9a**, flow rate and oxygen saturation have been predicted for different level of ventricular hypoplasia at different circulatory components (aorta, pulmonary artery, systemic veins, and left coronary arteries). On the right, dashed horizontal lines indicate critical levels of oxygen that threat life viability. **[NOUS ALLONS REFAIRE FAIRE LES FIGURES]** On **FIG. 9b** is represented the evolution of Pressure volume loop (PV-loop) from prenatal to after 2 minutes, 1 hour, 12 hours and neonatal circulation for a healthy subject.

**FIGs. 9a** and **9b** depict different scenarios of left heart hypoplasia. For a certain levels of the congenital malformation, the life of the patient is at risk since the oxygen saturation in the systemic veins, depicted by the horizontal dashed line in **FIG. 9a**, is below the physiological viable value. This evaluation shows an explicit physiological response to a combination of cardiac malformations, meaning that any set of measurements can be gathered through the digital twin and visualized with this type of physiological response. Nowadays, this physiological response is indirectly obtained by a set of measurements (morphological and functional), but different CHD classes cannot be compared, especially if different morphological and functional measurements are recorded. On contrary, this functional assessment allows a direct comparison of any CHD, as well as healthy patients.

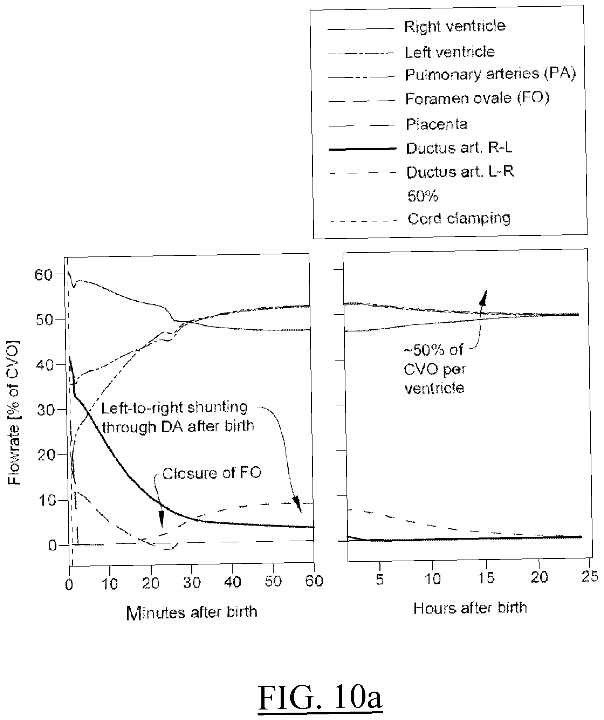

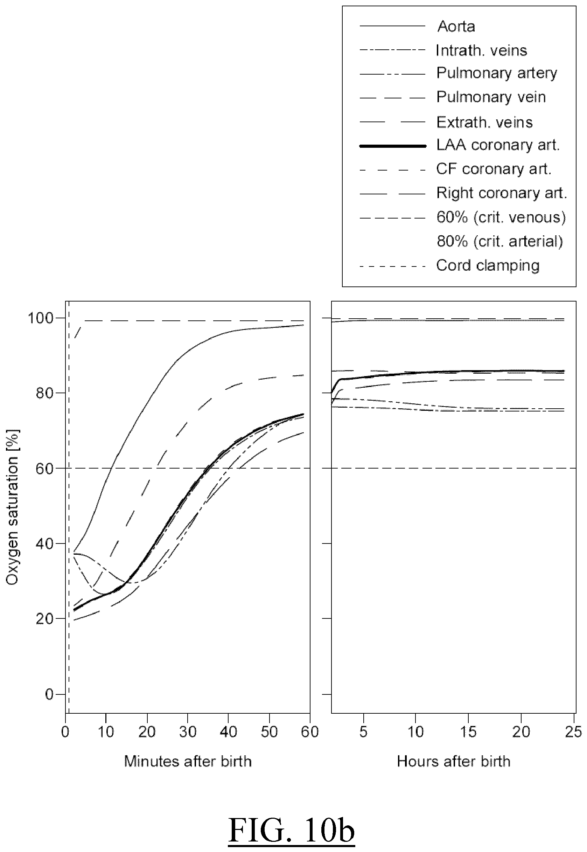

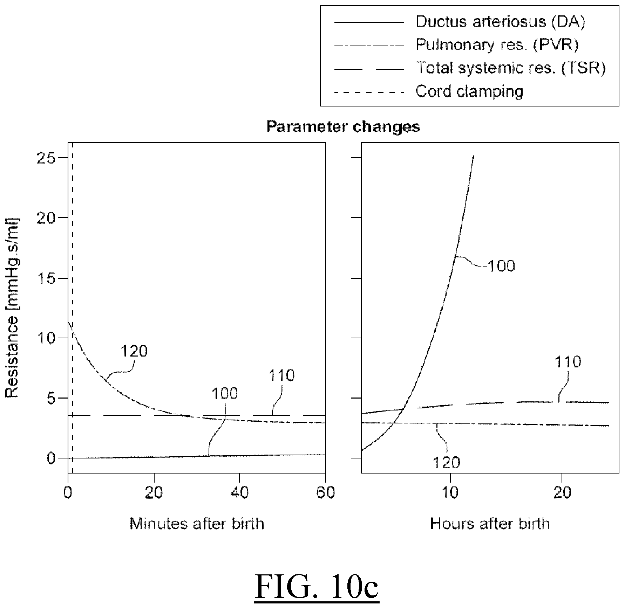

**FIG. 10a** shows the transition of the extracted biomarker flowrate during prenatal to neonatal transition, on the left for the first hour after the cord clamping, and on the right within the next hours after the birth. **FIG. 10a** shows the closure of the foramen ovale and the shunting through the ductus arteriosus (DA) that occur after birth in the case of a normal subject. **FIG. 10b** shows the transition of the extracted biomarker oxygen saturation during prenatal to neonatal transition in the case of a normal subject. **FIG. 10c** shows the evolution of the parameters ductus anteriosus (DA) **100**, pulmonary vascular resistance (PVR) **110** and total systemic resolution (TSR) **120** applied to the calibrated surrogate cardiovascular model of the fetus and the newborn during prenatal to neonatal transition.

Referring back to **FIG. 7**, it is now discussed a virtual intervention **G**. If the transition of the extracted biomarker(s) shows that the life of the subject is not viable or at risk after birth according to an inspection of the predicted postnatal condition **(E2)**, then virtual intervention may be simulated. The virtual modifications **G allow** either a modification of the prenatal digital twin by simulating an intervention at prenatal stage **(D1)** or a postnatal surgery **(E1)** or both in a sequential fashion. The method for predicting the cardiovascular behavior of a newborn to birth from his/her fetus physiological parameters is used for simulating a surgical intervention on a cardiovascular system of the fetus and/or the newborn. A physical change of the cardiovascular system of the fetus, result of the surgical intervention to be simulated, is inputted by modifying the calibrated surrogate cardiovascular model of the fetus. A physical change of the cardiovascular system of the newborn, result of the surgical intervention to be simulated, is inputted by modifying the calibrated surrogate cardiovascular model of the newborn. The modification on the calibrated surrogate cardiovascular model of the fetus and/or the modification on the calibrated surrogate cardiovascular model of the newborn having been inputted, the predicting E1 is carried out again. In examples, the extracting D2, E2 can be performed for computing a new transition of each of the extracted biomarkers.

Simulating a surgical intervention does not affect the real surgical intervention and has to be considered as a decision-support tool; the performance of the surgical intervention remaining at the free discretion of the physician.

An example of a prenatal intervention is an intrauterine angioplasty. An example of postnatal interventions is a Norwood procedure or a specific medication dose protocol to avoid the closure of the ductus arteriosus in HLHS patients. The use of the combined prenatal and postnatal digital twins allow a quantitative evaluation to correct the detected critical condition at fetal stage.

In a particular example, the present disclosure may comprise a computer-implemented method for predicting a cardiovascular behavior of a fetus from his/her physiological parameters, the method:
- obtaining (A) physiological parameters of the fetus, in particular physiological parameters inferred from medical images;
- obtaining (C1) a non-calibrated surrogate cardiovascular model modeling a cardiovascular system of the fetus and modelling at least one physiological change triggered by the birth;
- calibrating (C2) the non-calibrated surrogate cardiovascular model with a data assimilation algorithm using the obtained physiological parameters of the fetus on the non-calibrated surrogate cardiovascular model, thereby obtaining (D1) a calibrated surrogate cardiovascular model of the fetus;
- extracting (D2), from the calibrated surrogate cardiovascular model of the fetus, predicted biomarkers of the fetus, each of the predicted biomarker providing a quantitative assessment of the biomarker at a given point in time.

It is to be understood that examples that may apply to each of the blocks A, B, C1, C2 and D1 discussed in reference to FIG. 3 and 7 also apply.

It is to be understood that the present invention allows a reuse of the physiological parameters that were obtained after model calibration. Some physiological parameters can be extracted at prenatal stage and then they can be directly used at neonatal stage. Some of these parameters are easier to obtain at fetal stage, such as cerebral arterial flows.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

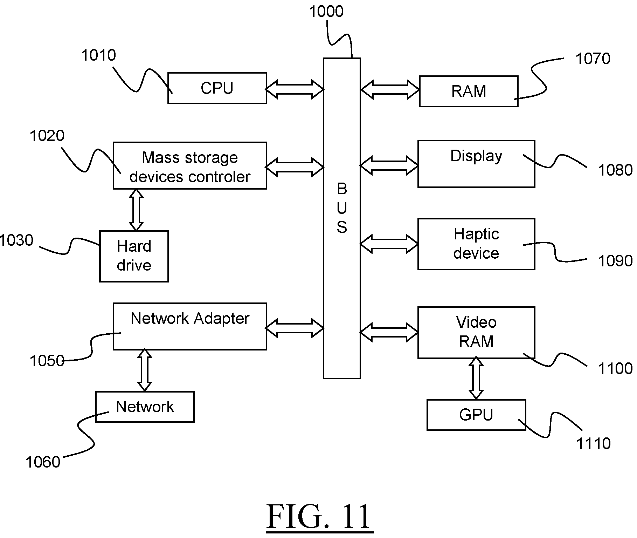

FIG. 11 shows an example of the system, wherein the system is a client computer system, e.g. a workstation of a user.

The client computer of the example comprises a central processing unit (CPU) 1010 connected to an internal communication BUS 1000, a random-access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for predicting a cardiovascular behavior of a newborn to birth from his/her fetus physiological parameters, the method comprising:
- obtaining (A) physiological parameters of the fetus, in particular physiological parameters inferred from medical images;
- obtaining (C1) a non-calibrated surrogate cardiovascular model modeling a cardiovascular system of the fetus and modelling at least one physiological change triggered by the birth;
- calibrating (C2) the non-calibrated surrogate cardiovascular model with a data assimilation algorithm using the obtained physiological parameters of the fetus on the non-calibrated surrogate cardiovascular model, thereby obtaining (D1) a calibrated surrogate cardiovascular model of the fetus;
- predicting (E1) the cardiovascular behavior of the newborn by triggering the at least one physiological change of the calibrated surrogate cardiovascular model of the fetus, thereby obtaining a calibrated surrogate cardiovascular model of the newborn.

2. The computer-implemented method of claim 1, wherein the at least one physiological change triggered by the birth is selected among:
- a clamping of a placenta of the newborn;
- a breathing of the newborn;
- a closure of a foramen ovale of the newborn; and/or
- a closure of a ductus arteriosus of the newborn.

3. The computer-implemented method of claim 1 or 2, further comprising, before the obtaining (C1):
- obtaining (B) a generic non-calibrated surrogate cardiovascular model modeling a cardiovascular system of a general population of fetuses;
and further comprising building the non-calibrated surrogate cardiovascular model modeling the cardiovascular system of the fetus by combining the obtained (B) generic non-calibrated surrogate cardiovascular model with the obtained (A) physiological parameters of the fetus, thereby the obtained non-calibrated surrogate cardiovascular model modeling the cardiovascular system of the fetus comprises at least all the obtained physiological parameters of the fetus.

4. The computer-implemented method of claim 3, wherein the general population of fetuses of the generic non-calibrated surrogate cardiovascular model comprises at least one cardiovascular behavior representative of a cardiovascular malformation, preferable one of the at least one cardiovascular behavior is representative of the cardiovascular malformation of the fetus.

5. The computer-implemented method of any one of claims 1 to 4, wherein the wherein the obtained non-calibrated surrogate cardiovascular model modeling a cardiovascular system of the fetus is a lumped-parameter model.

6. The computer-implemented method of any one of claims 1 to 5, wherein the data assimilation algorithm for calibrating (C1) is an Unscented Kalman Filter.

7. The computer-implemented method of any one of claims 1 to 6, further comprising:
- extracting (D2), from the calibrated surrogate cardiovascular model of the fetus, predicted biomarkers of the fetus, each of the predicted biomarker providing a quantitative assessment of the biomarker.

8. The computer-implemented method of any one of claims 1 to 7, further comprising:
- extracting (E2), from the calibrated surrogate cardiovascular model of the newborn, predicted biomarkers of the newborn, each of the predicted biomarker providing a quantitative assessment of the biomarker.

9. The computer-implemented method of claims 7 and 8, wherein further comprising:
- computing, from the extracted (D2) biomarkers of the fetus and the extracted (E2) biomarkers of the newborn, a transition of each of the extracted biomarkers.

10. The computer-implemented method of any one of claims 7 to 9, wherein the biomarkers are:
- morphological biomarkers such as a size of the fetus and/or the newborn, a cardiac frequency, a diameter of an aorta, a size of a left ventricle, a size of a right ventricle, a valve size; and/or
- functional biomarkers such as a blood flow velocity.

11. The computer-implemented method of any one of claims 1 to 10, wherein the non-calibrated surrogate cardiovascular model is a surrogate model, preferably the non-calibrated surrogate cardiovascular model is a 0D surrogate model.

12. A computer-implemented method of use of the method according to any one of claim 1 to 11, for simulating a surgical intervention on a cardiovascular system of the fetus and/or the newborn, comprising:
- inputting a physical change of the cardiovascular system of the fetus by modifying the calibrated surrogate cardiovascular model of the fetus and/or inputting a physical change of the cardiovascular system of the newborn by modifying the calibrated surrogate cardiovascular model of the newborn;
- performing again the predicting the cardiovascular behavior of the newborn.

13. A computer program comprising instructions for performing the method of any of claims 1 to 11 and/or claim 12.

14. A computer readable storage medium having recorded thereon a computer program of claim 13.

15. A system comprising a processor coupled to a memory and a graphical user interface, the memory having recorded thereon the computer program of claim 13.
